(19) Europäisches Patentamt
European Patent Office
Office européen des brevets

(11) Veröffentlichungsnummer: **0 330 966 B1**

# (12) EUROPÄISCHE PATENTSCHRIFT

(45) Veröffentlichungstag der Patentschrift: **30.03.94**

(51) Int. Cl.5: **C07D 251/34**, C08G 18/02, C08G 18/79, C08G 18/80, C09D 175/04

(21) Anmeldenummer: **89102962.1**

(22) Anmeldetag: **21.02.89**

## (54) Verfahren zur Herstellung von Isocyanuratgruppen aufweisenden Polyisocyanaten.

(30) Priorität: **27.02.88 DE 3806276**

(43) Veröffentlichungstag der Anmeldung:
**06.09.89 Patentblatt 89/36**

(45) Bekanntmachung des Hinweises auf die Patenterteilung:
**30.03.94 Patentblatt 94/13**

(84) Benannte Vertragsstaaten:
**BE DE ES FR GB IT NL**

(56) Entgegenhaltungen:
**EP-A- 0 010 589**
**FR-A- 1 566 256**

(73) Patentinhaber: **BAYER AG**

**D-51368 Leverkusen(DE)**

(72) Erfinder: **Pedain, Josef, Dr.**
**Haferkamp 6**
**D-5000 Köln 80(DE)**
Erfinder: **Bock, Manfred, Dr.**
**311 Myrtle Lane**
**Sewickley, Pa 15143(US)**
Erfinder: **Dieris, Carl-Gerd, Dr.**
**Andreasstrasse 5b**
**D-4047 Dormagen 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft ein neues Verfahren zur Herstellung von Isocyanuratgruppen aufweisenden Polyisocyanaten durch Trimerisierung eines Teils der Isocyanatgruppen von Hexamethylendiisocyanat (nachstehend "HDI" genannt).

Die Verwendung von quaternären Ammoniumhydroxiden als Katalysator für die Trimerisierung von Isocyanatgruppen ist bekannt und mehrach vorbeschrieben. So werden gemäß JP-PS 601 337 (US-A- 3 487 080, FR-A- 1566256) quaternäre Ammoniumhydroxide zusammen mit bestimmten Cokatalysatoren verwendet, wobei in den Ausführungsbeispielen vor allem die Teiltrimerisierung von aromatischen Diisocyanaten gezeigt wird. Die Teiltrimerisierung von HDI wird in einem konkreten Ausführungsbeispiel gezeigt.

Benutzt man die Katalysatoren, die in den Beispielen dieser Patentschrift zur Trimerisierung benutzt werden zur Umsetzung von HDI und verfährt wie dort angegeben, so entstehen überwiegend getrübte, für Lackanwendungen unbrauchbare Produkte.

Das Verfahren der EP-A-10 589 stellt eine Weiterentwicklung des Verfahrens der genannten japanischen Patentschrift dar. Gemäß dieser Vorveröffentlichung werden als Katalysatoren für die Trimerisierung von HDI Hydroxyalkyl-Substituenten aufweisende quaternäre Ammoniumhydroxide verwendet. HDI läßt sich hiermit ausgezeichnet trübungsfrei trimerisieren. Nachteilig bei diesem Verfahren ist, daß sich die Hydroxyalkylammoniumhydroxide nur sehr schwierig farblos herstellen lassen und in verhältnismäßig großen Mengen bis 0,6 % angewendet werden müssen. Die Verfahrensprodukte, d.h. die von überschüssigem Ausgangsdiisocyanat befreiten, Isocyanuratgruppen aufweisenden Polyisocyanate können daher unter Umständen eine gelbliche Färbung aufweisen.

Die EP-A-47 452 beschreibt die Herstellung von Mischtrimerisaten auf Basis von HDI und IPDI, wobei keine von Kohlendioxid weitgehend befreiten Ausgangsdiisocyanaten eingesetzt und demzufolge ebenfalls, wie aus den Ausführungsbeispielen ersichtlich, vergleichsweise hohe Mengen an Katalysatoren verwendet werden.

Andere bekannte Verfahren zur Herstellung von Isocyanuratgruppen aufweisenden Polyisocyanaten auf Basis von HDI haben ebenfalls schwerwiegende Nachteile. So werden beispielsweise in GB-A- 920 080, DE-A- 3 100 262, DE-A-3 219 608 oder DE-A- 3 240 613 Verfahren zur Trimerisierung von HDI unter Verwendung von metallhaltigen Katalysatoren und von Cokatalysatoren wie Phenolen, Alkoholen oder tert. Aminen beschrieben. Die Metallverbindungen lassen sich aus den Endprodukten nicht oder nur mittels sehr aufwendigen Verfahren entfernen und können spätere Anwendungen ebenso wie die Haltbarkeit der Verfahrensprodukte empfindlich stören. Im übrigen führt die Verwendung von aktive Wasserstoffatome aufweisenden Cokatalysatoren zu Nebenreaktionen unter Verbrauch wertvoller Isocyanatgruppen. Dies gilt auch für das Verfahren der EP-A-155 559, bei welchem Ammoniumsalze von organischen Säuren als Katalysator unter gleichzeitiger Mitverwendung von hohen Anteilen an alkoholischen Verbindungen eingesetzt werden.

Bei den Verfahren der EP-A-57 653, EP-A-89 297 Und EP-A-187 105 werden siliziumorganische Katalysatoren in vergleichsweise hohen Mengen eingesetzt. Auch diese Verbindungen können nicht restlos aus dem Verfahrensprodukt entfernt werden und bei dessen Verwendung zu Störungen führen.

Es war daher die der Erfindung zugrundeliegende Aufgabe, ein neues Verfahren zur teilweisen Trimerisierung der Isocyanatgruppen von HDI zur Verfügung zu stellen, welches folgende Vorteile in sich vereinigt:

- Die Verfahrensprodukte sollten praktisch farblos sein, d.h. eine Farbzahl (HAZEN) nach DIN 53 409 von unter 100 aufweisen.
- Die Verfahrensprodukte sollten frei von Trübungen sein und sich in den üblichen Lacklösungsmitteln trübungsfrei lösen.
- Die Verfahrensprodukte sollten keine Metallionen enthalten.
- Das Verfahren sollte unter Verwendung von minimalen Mengen an Katalysatoren durchführbar sein, ohne auf die Mitverwendung von hohen Mengen an Isocyanatgruppen verbrauchenden Cokatalysatoren angewiesen zu sein.

Wie jetzt überraschenderweise gefunden wurde, konnte diese Aufgabe mit dem nachstehend näher beschriebenen erfindungsgemäßen Verfahren gelöst werden.

Gegenstand der Erfindung ist ein Verfahren zur Herstellung von Isocyanuratgruppen aufweisenden Polyisocyonaten durch Trimerisierung eines Teils der Isocyanatgruppen von Hexamethylendiisocyanat unter Verwendung von quaternären Ammoniumhydroxiden, der allgemeinen Formel

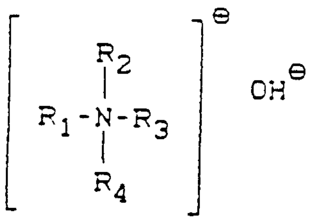

für welche
$R_1$, $R_2$ und $R_3$ für gleiche oder verschiedene Alkylreste mit 1 bis 18 Kohlenstoffatomen und $R_4$ für einen Benzyl-, 2-Hydroxyethyl-, 2-Hydroxypropyl- oder 2-Hydroxybutyl-Rest steht, als Trimerisierungskatalysator, Beendigung der Trimerisierungsreaktion beim angestrebten Trimerisierungsgrad durch Zugabe eines Katalysatorengifts und/oder thermische Desaktivierung des Katalysators und Entfernung von nicht umgesetztem Hexamethylendiisocyanat bis auf einen Restgehalt von max. 0,2 Gew.-%, dadurch gekennzeichnet, daß man

a) das als Ausgangsmaterial zum Einsatz gelangende Hexamethylendiisocyanat von Kohlendioxid bis auf einen Restgehalt von weniger als 20 ppm (Gewicht) befreit und

b) den Katalysator in einer Menge von weniger als 0,03 Gew.-%, bezogen auf das Gewicht des eingesetzten Hexamethylendiisocyanats, verwendet.

Von erfindungswesentlicher Bedeutung ist die Verwendung von praktisch Kohlendioxid-freiem HDI als Ausgangsmaterial. Das erfindungsgemäß zum Einsatz gelangende HDI weist einen Gehalt an Kohlendioxid von weniger als 20 ppm (Gewicht), vorzugsweise von weniger als 10 ppm (Gewicht) und besonders bevorzugt von weniger als 5 ppm (Gewicht) auf.

Technisches, durch Destillation gereinigtes HDI, wie es bislang auch zur Herstellung von Isocyanuratgruppen aufweisenden Polyisocyanaten eingesetzt wurde, weist beträchtliche Mengen (ca. 20 ppm bis 100 ppm (Gewicht)) an Kohlendioxid auf. Kohlendioxid kann bei der Herstellung in das HDI gelangen, beispielsweise bei der Phosgenierung von Kohlensäuresalzen des Hexamethylendiamins.

Es kann beim Lagern aus der Luft aufgenommen werden, und es kann durch chemische Reaktion der NCO-Gruppen, z.B. durch Carbodiimidbildung oder mit einer Spur Feuchtigkeit, entstehen. Frisch durch Vakuumdestillation gerelnigtes HDI enthält nach 24 Stunden im verschlossenen Gefäß beispielsweise 40 ppm Kohlendioxid. Über einen Zeitraum von ca. 6 Monaten gelagertes HDI kann, falls das Gebinde während der Lagerzeit geöffnet wurde, bis zu 0,6 Gew.-% Kohlendioxid enthalten.

Die Entfernung von Kohlendioxid aus HDI kann durch Ausblasen mit Reinstickstoff oder Edelgas, zum Beispiel mit Argon, erfolgen, z.B. bei 0 - 70 °C. Auch eine höhere Temperatur kann angewendet werden, bietet aber keine entscheidenden Vorteile. Auch mit einer Destillation am Stickstoff- oder Edelgasstrom kann Kohlendioxid entfernt werden. Die Art der Entfernung von Kohlendioxid ist nicht entscheidend für die Durchführung des erfindungsgemäßen Verfahrens. Allerdings ist durch eine einfache Destillation eine praktisch restlose Entfernung von Kohlendioxid im allgemeinen nicht möglich.

Beim erfindungsgemäßen Verfahren werden quaternäre Ammoniumhydroxide der vorstehend genannten allgemeinen Formel als Katalysator eingesetzt. Zu den bevorzugten Katalysatoren gehören solche, für welche
$R_1$, $R_2$ und $R_3$ für gleiche oder verschiedene Alkylreste mit 1 bis 4 Kohlenstoffatomen, besonders bevorzugt für Methylgruppen stehen und
$R_4$ die vorstehend genannte Bedeutung hat.

Besonders bevorzugte Katalysatoren sind N,N,N-Trimethyl-N-benzylammoniumhydroxid und N,N,N-Trimethyl-N-(2-hydroxypropyl)-ammoniumhydroxid.

Die erfindungsgemäß einzusetzenden quaternären Ammoniumhydroxide bzw. ihre Herstellung sind bekannt. Sie sind als technische Produkte teilweise als farblose Substanzen oder Lösungen verfügbar, teilweise aber auch - wie bereits oben ausgeführt - nur als intensiv gefärbte Lösungen erhältlich. Diese Eigenfarbe der Katalysatoren wirkt sich bei den Verfahren des obengenannten Standes der Technik zur Herstellung von Isocyanuratgruppen aufweisenden Polyisocyanaten auf Basis von HDI oftmals nachteilig aus, da bei den vorbekannten Verfahren des Standes der Technik die Katalysatoren in vergleichsweise hohen Mengen eingesetzt werden müssen. Beim erfindungsgemäßen Verfahren spielt diese Eigenfarbe der Katalysatoren dagegen keine Rolle, da der Katalysator nur in äußerst geringen Konzentrationen zum Einsatz gelangt.

Die Menge des eingesetzten Katalysators liegt beim erfindungsgemäßen Verfahren, bezogen auf zum Einsatz gelangendes HDI, bei weniger als 0,03 Gew.-%. Vorzugsweise werden weniger als 0,01 Gew.-%

und besonders bevorzugt 0,0005 bis 0,005 Gew.-% des Katalysators, bezogen auf eingesetztes HDI, verwendet. Die jeweils optimale Menge an Katalysator hängt von der Natur der eingesetzten quaternären Ammoniumverbindung ab und kann mit Hilfe eines orientierenden Vorversuchs leicht ermittelt werden. Im Falle der Verwendung von N,N,N-Trimethyl-N-benzylammoniumhydroxid sind besonders niedrige Mengen im Rahmen der oben gemachten Ausführungen ausreichend.

Im Unterschied zu dem Verfahren der JP-PS 601 337 sind beim erfindungsgemäßen Verfahren keine Cokatalysatoren erforderlich. Insbesondere kann auf die Mitverwendung von größeren Mengen an Verbindungen mit gegenüber Isocyanatgruppen reaktionsfähigen Gruppen wie Phenole, Oxime und insbesondere Methanol verzichtet werden, wodurch Nebenreaktionen zwischen einem Teil der Isocyanatgruppen des Ausgangsdiisocyanats und diesen Reaktivgruppen unter Verbrauch von wertvollen Isocyanatgruppen ebenso vermieden werden kann, wie die Bildung von Trübungen, die auf diese Nebenprodukte zurückzuführen sind.

Das erfindungsgemäße Trimerisierungsverfahren ist somit auch sehr gut durchführbar, wenn auf die Ausbildung von Urethangruppen bei der Katalyse verzichtet wird. Da aber viele der beim erfindungsgemäßen Verfahren eingesetzten Katalysatoren in Hydroxylgruppen enthaltenden Lösungsmitteln gelöst sind oder selbst Hydroxylgruppen tragen, wird die Bildung von Urethangruppen beim erfindungsgemäßen Verfahren nicht ausgeschlossen. Besonders bevorzugt ist jedoch die Verwendung von solchen Lösungsmitteln für die Katalysatoren, die keine gegenüber Isocyanatgruppen reaktionsfähigen Gruppen aufweisen. Die Katalysatoren können auch völlig lösungsmittelfrei verwendet werden.

Im Falle der Verwendung von Hydroxylgruppen aufweisenden Lösungsmitteln werden vorzugsweise solche eingesetzt, die mit HDI keine bei Raumtemperatur festen Umsetzungsprodukte bilden und die Functionalität der Verfahrensprodukte möglichst wenig herabsetzen. Derartige, Hydroxylgruppen aufweisende Lösungsmittel sind beispielsweise 2-Ethylhexandiol-1,3 oder 2-Ethylhexanol. Gut geeignete Lösungsmittel ohne gegenüber Isocyanatgruppen reaktionsfähige Gruppen sind beispielsweise Dimethylformamid, Dimethylacetamid, Dimethylsulfoxid und Acetonitril.

Wegen der äußerst geringen Katalysatormengen ist die Dosierung und Einmischung des reinen, unverdünnten Katalysators insbesondere bei kontinuierlicher großtechnischer Arbeitsweise zwar möglich jedoch nicht einfach. Es ist daher bevorzugt, mit stark verdünnten Katalysatorlösungen zu arbeiten, wobei Konzentrationen von unter 5 Gew.-%, vorzugsweise von unter 1 Gew.-% besonders gut geeignet sind. Zur Herstellung derartiger Katalysatorlösungen ist es oftmals angezeigt, in den Katalysatoren vorliegende Lösungsmittel, insbesondere das schädliche Methanol durch schonende Destillation nach Zusatz eines geeigneten Lösungsmittels der oben beispielhaft genannten Art zu entfernen.

Die Trimerisierungsreaktion wird vorzugsweise, vom Lösungsmittel für den Katalysator abgesehen, lösungsmittelfrei durchgeführt, was die Mitverwendung von üblichen Lacklösungsmitteln bei der Trimerisierungsreaktion, beispielsweise von Estern wie Butylacetat oder Ethoxyethylacetat, Ketonen wie Methylisobutyl-keton oder Methylethylketon oder Kohlenwasserstoffen wie Xylol bzw. von Gemischen derartiger Lösungsmitteln jedoch nicht ausschließt. Da im Anschluß an die Trimerisierungsreaktion jedoch nicht umgesetztes HDI entfernt wird, würde die Mitverwendung derartiger Lösungsmittel während der Trimerisierungsreaktion lediglich auf einen überflüssigen, zusätzlichen Aufwand hinauslaufen.

Zur Durchführung der Trimerisierungsreaktion wird das als Ausgangsmaterial eingesetzte, vom Kohlendioxid weitgehend befreite HDI mit dem Katalysator versetzt, wobei die Katalysatorzugabe auch portionsweise im Verlauf der Trimerisierungsreaktion erfolgen kann. Die Trimerisierungsreaktion wird im allgemeinen innerhalb des Temperaturbereichs von 0 °C bis 100 °C, vorzugsweise 20 bis 80 °C durchgeführt und vorzugsweise bei Erreichen eines Trimerisierungsgrads von 10 bis 40, vorzugsweise 20 bis 30 % abgebrochen. Unter "Trimerisierungsgrad" ist hierbei der Prozentsatz der im Ausgangsdiisocyanat vorliegenden Isocyanatgruppen zu verstehen, die im Sinne einer Trimerisierungsreaktion abreagieren.

Zur Beendigung der Trimerisierungsreaktion wird im allgemeinen dem Reaktionsgemisch ein geeignetes Katalysatorgift zugefügt. Geeignete Katalysatorgifte sind beispielsweise anorganische Säuren wie Salzsäure, phosphorige Säure oder Phosphorsäure, Sulfonsäuren oder ihre Derivate wie Methansulfonsäure, p-Toluolsulfonsäure, p-Toluolsulfonsäuremethyl- oder -ethylester oder perfluorierte Sulfonsäuren wie beispielsweise Nonafluorbutansulfonsäure. Besonders gut geeignete Desaktivatoren, d.h. Katalysatorengifte sind saure Ester der phosphorigen Säure oder der Phosphorsäure wie beispielsweise Dibutylphosphit, Dibutylphosphat oder Di-(2-ethylhexyl)-phosphat, die vorzugsweise in Form einer verdünnten Lösung in HDI eingesetzt werden. Die Desaktivatoren werden dem Reaktionsgemisch im allgemeinen in einer dem Katalysator zumindest äquivalenten Menge zugefügt. Da sich die Katalysatoren jedoch während der Trimerisierungsreaktion teilweise zersetzen können, ist oftmals die Zugabe einer unter äquivalenten Menge des Desaktivators ausreichend. Bei Verwendung von thermisch labilen Katalysatoren, beispielsweise von quaternären Ammoniumhydroxiden mit Hydroxyalkyl-Substituenten am Stickstoff, kann oftmals auch auf die

Zugabe eines Katalysatorengifts völlig verzichtet werden, da bei Verwendung derartiger Katalysatoren oftmals ein Abbruch der Reaktion durch kurzzeitiges Erhitzen des Reaktionsgemischs auf oberhalb 100 ° C liegende Temperaturen ausreicht (thermische Zersetzung, d.h. Desaktivierung des Katalysaotrs). Andererseits ist zwecks Gewährleistung einer sicheren Abstoppung der Reaktion oftmals auch die Verwendung einer größeren als der äquivalenten Menge, beispielsweise einer doppelt äquivalenten Menge des Desaktivators zweckmäßig. Vorzugsweise wird somit unter Verwendung von Desaktivatoren (Katalysatorengiften) in bis zu 2-fach äquivalenten Mengen, bezogen auf die Menge des eingesetzten Katalysators, gearbeitet.

Nach der Desaktivierung wird überschüssiges HDI durch eine geeignete Maßnahme wie z.B. Extraktion (beispielsweise unter Verwendung von n-Hexan als Extraktionsmittel) oder vorzugsweise Dünnschichtdestillation im Hochvakuum bis auf einen Restgehalt von maximal 0,2 Gew.-%, vorzugsweise von weniger als 0,1 Gew.-% freies HDI entfernt.

Die erfindungsgemäßen Verfahrensprodukte stellen farblose Flüssigkeiten einer Farbzahl (HAZEN) nach DIN 53 409 von unter 100, im allgemeinen von unter 50 eines Isocyanatgehalts von 10 bis 24 Gew.-% und einer Viskosität bei 23 ° C von 500 bis 10.000 mPa.s dar.

Da bei der Durchführung des erfindungsgemäßen Verfahrens nur äußerst geringe Katalysatormengen eingesetzt werden, kann auch die Menge des Desaktivators, d.h. des Katalysatorengifts entsprechend gering gehalten werden, was zur Folge hat, daß die erfindungsgemäßen Verfahrensprodukte nur äußerst geringe Mengen an Nebenprodukten, die aus Katalysator und Katalaysatorgift entstehen, enthalten, die gelöst bleiben und bei der späteren Verwendung der Verfahrensprodukte nicht stören. Auch bei Verwendung von technischem HDI, welches nicht vorab durch Destillation über schwach basischen Verbindungen wie Metalloxiden oder Natriumhydrogencarbonat einer üblichen Reinigungsoperation zwecks Entfernung von Spuren an chlorhaltigen Verbindungen unterzogen worden ist, werden klare und farblose Verfahrensprodukte erhalten. Wegen ihrer niedrigen Viskosität sind die Verfahrensprodukte insbesondere zur Herstellung von lösungsmittelfreien oder lösungsmittelarmen Zweikomponenten-Polyurethanlacken geeignet.

Bei der erfindungsgemäßen Verwendung der erfindungsgemäßen Verfahrensprodukte können diese in mit Blockierungsmitteln für Isocyanatgruppen blockierter Form verwendet werden. Geeignete Blockierungsmittel sind beispielsweise die in EP-A-10 589, Seite 15, Zeilen 14-26 beispielhaft genannten Verbindungen.

Die erfindungsgemäßen Verfahrensprodukte dienen insbesondere in Kombination mit den aus der Polyurethanlacktechnik bekannten Polyhydroxypolyestern, Polyhydroxypolyethern und insbesondere Polyhydroxypolyacrylaten zur Herstellung von hochwertigen Zweikomponenten-Polyurethanlacken, wobei diese Lacke neben den genannten höhermolekularen Polyhydroxylverbindungen auch niedermolekulare, vorzugsweise aliphatische Polyole enthalten können. Insbesondere Kombinationen der erfindungsgemäßen Verfahrensprodukte mit Polyhydroxypolyacrylaten stellen wertvolle Zweikomponenten-Bindemittel für hochwertige, äußerst wetterbeständige Autoreparaturlacke dar.

Auch Polyamine, insbesondere in blockierter Form als Polyketimine oder Oxazolidine sind denkbare Reaktionspartner für die erfindungsgemäßen Verfahrensprodukte.

Die Mengenverhältnisse, in welchen die erfindungsgemäßen, gegebenenfalls blockierten Polyisocyanate und die genannten Reaktionspartner bei der Herstellung von Polyurethanlacken umgesetzt werden, werden im allgemeinen so gewählt, daß auf eine (gegebenenfalls blockierte) Isocyanatgruppe 0,8-3, vorzugsweise 0,9-1,8, Hydroxyl-, Amino- und/oder Carboxylgruppen entfallen.

Zur Beschleunigung der Aushärtung können in bekannter Weise die in der Isocyanatchemie üblichen Katalysatoren verwendet werden, wie z.B. tert.- Amine wie Triethylamin, Pyridin, Methylpyridin, Benzyldimethylamin, N,N-Dimethylaminocyclohexan, N-Methylpiperidin, Pentamethyldiethylentriamin, N,N'-Endoethylenpiperazin, N,N'-Dimethylpiperazin oder Metallsalze wie Eisen(III)-chlorid, Zinkchlorid, Zink-2-ethylcaproat, Zinn(II)-2-ethylcaproat, Dibutylzinn(IV)-dilaurat oder Molybdänglykolat.

In blockierter Form werden die erfindungsgemäßen Verfahrensprodukte in Kombination mit Polyhydroxylverbindungen der genannten Art insbesondere zur Herstellung von Einbrennlacken verwendet, die in Abhängigkeit vom eingesetzten Blockierungsmittel bei Temperaturen von 80 bis 180 ° C zu hochwertigen Lacküberzügen ausgehärtet werden können.

Zur Herstellung der gebrauchsfertigen Lacke werden gegebenenfalls blockiertes Polyisocyanat, polyfunktioneller Reaktionspartner, gegebenenfalls Katalysator für die Isocyanat-Polyaddition und die üblichen Zusätze, wie z.B. Pigmente, Farbstoffe, Füllstoffe, und Verlaufmitel miteinander auf einem üblichen Mischaggregat, wie z.B. auf einer Sandmühle entweder mit oder ohne Lösungs- und Verdünnungsmittel gut vermischt und homogenisiert.

Die Anstrich- und Überzugsmittel können in Lösung oder aus der Schmelze, oder in fester Form nach den üblichen Methoden wie z.B. Streichen, Rollen, Gießen, Spritzen, dem Wirbelsinterverfahren oder dem elektrostatischen Pulversprühverfahren auf den zu beschichtenden Gegenstand aufgebracht werden.

Die die erfindungsgemäßen Polyisocyanate enthaltenden Lacke ergeben Filme, die überraschend gut auf metallischem Untergrund haften, besonders lichtecht, wärmefarbstabil und sehr abriebfest sind. Darüber hinaus zeichnen sie sich durch große Härte, Elastizität, sehr gute Chemikalienbeständigkeit, hohen Glanz, ausgezeichnete Wetterbeständigkeit und gute Pigmentierbarkeit aus.

In den nachfolgenden Beispielen beziehen sich alle Prozentangaben auf Gewichtsprozente.

Beispiel 1 (Herstellung einer Katalysatorlösung)

1000 g einer handelsüblichen, farblosen Lösung von N,N,N-Trimethyl-N-benzylammoniumhydroxid, 40 %ig in Methanol, werden mit 600 g 2-Ethylhexandiol-1,3 versetzt und verrührt. Unter gutem Rühren wird vom Methanol bei 30 bis 40 °C im Vakuum der Wasserstrahlpumpe restlos entfernt. Die 40 %ige Stammlösung wird mit weiterem 2-Ethylhexandiol-1,3 auf eine Wirkstoffkonzentration von 0,5 % eingestellt (I).

Beispiel 2 (Herstellung einer Katalysatorlösung)

Man verfährt wie in Beispiel 1 benutzt aber anstelle von 2-Ethylhexandiol-1,3 Dimethylformamid zum Austausch des Methanols und zum weiteren Verdünnen.

Man erhält eine 0,5 %ige Wirkstoff-Lösung in Dimethylformamid (II).

Beispiel 3 (Herstellung einer Katalysatorlösung)

100 g einer 70 %igen Lösung in Methanol von N,N,N-Trimethyl-N-(2-hydroxypropyl)-ammoniumhydroxid, hergestellt durch Umsetzung von Trimethylamin mit Propylenoxid in Methanol werden mit 60 g 2-Ethylhexanol versetzt und im Vakuum der Wasserstrahlpumpe von Methanol befreit. Mit weiterem 2-Ethylhexanol wird auf eine Wirkstoffkonzentration von 4 % eingestellt (III). Die Lösung ist braun gefärbt.

Beispiel 4 (erfindungsgemeäß)

3200 g HDI werden bei ca. 20 °C in einem Rührgefäß durch Anlegen von Vakuum (50 mbar) und kräftiges Rühren in ca. 10 Minuten entgast. Der Gasraum der Apparatur wird dann mit reinem Stickstoffgefüllt. Anschließend wird ein starker Strom von reinem, trockenem Stickstoff etwa 1 Stunde bei ca. 25 °C durch die Flüssigkeit geleitet. Während das eingesetzte HDI einen $CO_2$-Gehalt von 44 ppm zeigte, war nach den genannten Maßnahmen der $CO_2$-Gehalt auf 2 ppm abgefallen. Während der gesamten weiteren Reaktion wird weiter Stickstoff durch die Reaktionsmasse geleitet.

Anschließend werden 32 g (0,96 mMol der Base) der Katalysator-Lösung (I) innerhalb eines Zeitraums von 15 bis 30 Minuten eingetropft und dann innerhalb von 30 Minuten auf 60 °C erhitzt. Die Reaktion verläuft jetzt leicht exotherm, der Gefäßinhalt wird durch Kühlen auf 60 bis 65 °C gehalten. Nach ca. 0,5 h klingt die Reaktion ab. Der NCO-Gehalt des Rohprodukts liegt dann bei 42 %. Man rührt jetzt nach etwa 1 h bei 60 °C nach bis ein NCO-Gehalt von 38 % erreicht ist. Dann wird mit 0,32 g einer 25 %igen Lösung von Dibutylphosphat (0,38 mMol) in HDI abgestoppt und 15 Minuten nachgerührt. Man läßt anschließend die Flüssigkeit auf Umgebungstemperatur abkühlen und entfernt überschüssiges HDI durch eine Dünnschichtdestillation.

Man erhält 1382 g eines klaren hellen Polyisocyanats, das durch folgende Daten charakterisiert wird:

Viskosität: 1800 mPa.s/23 ° C
Farbzahl (Hazen) nach DIN 53 409: 20
NCO-Gehalt: 22,3 %
Gehalt an freiem HDI: 0,05 %
Verdünnbarkeit mit Xylol: Gegeben ohne Eintrübung bis unter einen Feststoffgehalt von 10 %

Beispiel 5 (Vergleichsversuch)

Man verfährt wie in Beispiel 4 angegeben und legt 3200 g HDI in einer Apparatur vor, entgast dieses durch Anlegen von Vakuum und füllt die Apparatur mit Stickstoff. Man verzichtet aber anschließend auf das Durchblasen von Stickstoff und auch in der Folge wird in die Flüssigkeit nicht Stickstoff eingeleitet sondern nur übergeleitet. Der $CO_2$-Gehalt des HDI hat sich durch dise Maßnahme nur unwesentlich erniedrigt. Er liegt bei 38 ppm.

Dann fügt man wie in Beispiel 1 angegeben 32 g der Katalysatorlösung (I) zu und erhitzt auf 60 °C. Dabei ist keine Reaktion zu beobachten, der NCO-Gehalt sinkt kaum ab. Man gibt nach 4 h bei 60 °C erneut 32 g Katalysatorlösung zu, die Reaktion springt noch immer nicht an. Nach weiterer Zugabe von 64 g Katalysator setzt eine Reaktion ein, die man bei einem NCO-Gehalt von 38,2 % durch Zugabe von 1,58 g einer 25 %igen Lösung von Dibutylphosphat abbricht. Beim Abkühlen auf 25 ° C zeigt sich die Lösung als trübe Flüssigkeit aus der sich nach und nach ein weißer Niederschlag abscheidet. Nach Entfernen von freiem HDI durch Dünnschichtdestillation erhält man ein trübes gelbes Verfahrensprodukt mit einem NCO-Gehalt von 21,4 %. Beim Verdünnen mit Butylacetat löst sich die Trübung nicht auf. Beim Verdünnen mit Xylol wird die Trübung noch verstärkt. Das Produkt ist für die Anwendung in hochwertigen PUR-Lacken unbrauchbar,

Beispiel 6 (erfindungsgemäß)

798 g frisch destilliertes Hexamethylendiisocyanat werden bei 20 ° C in einem Rührgefäß im Vakuum (<50 mbar) 30 Minuten kräftig gerührt. Der Gasraum der Apparatur wird dann mit Reinstickstoff gefüllt. Die Bestimmung von Kohlendioxid im HDI ergibt danach einen Gehalt von 44 ppm. Anschließend wird ein starker Strom von reinem, trockenem Stickstoff 1 Stunde bei 30 bis 40 ° C durch die Flüssigkeit geleitet. Die erneute Bestimmung des Gehaltes an $CO_2$ ergibt 2 ppm.

Während der Dauer der weiteren Reaktion wird trockener Stickstoff durch die Reaktionsmasse geleitet. Zur Einleitung der Trimerisierungsreaktion gibt man 12 g Katalysatorlösung II in ca. 30 Minuten tropfenweise zu und erhitzt langsam auf 70 ° C. Die Reaktion verläuft exotherm und kann ohne weitere Wärmezufuhr 1 Stunde bei ca. 75 ° C gehalten werden. Danach tropft man noch weitere 12 g Katalysatorlösung ein. Man läßt unter gutem Rühren noch weitere 30 Minuten reagieren und beendet die Umsetzung bei einem NCO-Gehalt von 42,4 % durch Zugabe von 0,6 g (Äquivalentverhältnis Katalysator: Abstopper = ca. 1:1) einer 25 %igen Lösung von Dibutylphosphat in HDI. Nach 15 Minuten wird die Flüssigkeit zur Abtrennung von Lösemittel (aus dem Katalysator) und HDI bei 130 ° C einer Dünnschichtdestillation unterworfen. Man erhält 230 g eines Polyisocyanats, das durch folgende Daten charakterisiert wird:
Viskosität: 2200 mPas/23 ° C.
NCO-Gehalt: 22,0 %
Gehalt an freiem HDI: 0,09 %
Farbzahl: 30 (DIN 53 409)

Beispiel 7 (erfindungsgemäß)

Man verfährt wie in Beispiel 6 und führt die Umsetzung bis zu einem NCO-Gehalt von 38,0 %. Nach dem Abstoppen der Reaktion und nach Dünnschichtdestillation bei 120 ° C erhält man 350 g eines Produktes mit folgenden Charakteristika:
Viskosität: 3.000 mPas/23 ° C
NCO-Gehalt: 21,7 %
Farbzahl: 40 (DIN 53 409)
Gehalt an freiem HDI: 0,1 %.

Beispiel 8 - 12 (erfindungsgemäß)

Es wird verfahren wie in Beispiel 1 angegeben, indem $CO_2$ durch Ausblasen mit einem starken Stickstoffstrom bei 40 bis 50 ° C aus dem HDI entfernt wird. Die weiteren Bedingungen der Polymerisierungsreaktion sind in Tabelle 1 angegeben. Die Desaktivierung des Katalysators erfolgt mit einer Ausnahme (Beispiel 10) mit Dibutylphosphat. Angegeben ist weiterhin der NCO-Gehalt der Reaktionsmasse, bei dem die Unterbrechung der Polymerisierungsreaktion eingeleitet wurde. Tabelle 2 gibt die charakteristischen Daten des Endprodukts nach Entfernung von überschüssigem HDI wieder.

Tabelle 1

| Beisp. | HDI (g) | $CO_2$-Gehalt nach Ausblasen mit $N_2$ | Katalysatorlösung aus Beisp. 3 | Katalysatorlösung aus Beisp. 1 | Desaktivierung thermisch oder mit Dibutylphospat (25 %ige Lösung in HDI) | NCO-Gehalt der Rohlösung |
|---|---|---|---|---|---|---|
| 8 | 3000 | 4 ppm | 12,4 g | - | 2 g bei 50° C | 39,2 % |
| 9 | 3000 | 6 ppm | 12,4 g | - | 0,5 g bei 60° C | 34,5 % |
| 10 | 2500 | 2 ppm | 7 g | - | durch Erhitzen auf 120° C | 41,3 % |
| 11 | 2500 | 2 ppm | - | 11,9 g | 0,13 g bei 60° C | 42,0% |
| 12 | 2500 | 10 ppm | - | 20 g | 0,52 g bei 70° C | 38,1 % |

Tabelle 2

| Beisp. | Produktmenge nach Entfernung von HDI | NCO-Gehalt | Viskosität 23° C/mPas | Farbzahl (Hazen) nach DIN 53 409 | Gehalt an freiem HDI |
|---|---|---|---|---|---|
| 8 | 1296 g | 22,8 % | 1700 | 70 | 0,12 % |
| 9 | 1560 g | 20,2 % | 9800 | 90 | 0,09 % |
| 10 | 880 g | 23,2 % | 1800 | 70 | 0,05 % |
| 11 | 805 g | 23,7 % | 1500 | 30 | 0,05 % |
| 12 | 1200 g | 22,0 % | 2100 | 40 | 0,05 % |

## Patentansprüche

1. Verfahren zur Herstellung von Isocyanuratgruppen aufweisenden Polyisocyanaten durch Trimerisierung eines Teils der Isocyanatgruppen von Hexamethylendiisocyanat unter Verwendung von quatemären

Ammnoniumhydroxiden der allgemeinen Formel

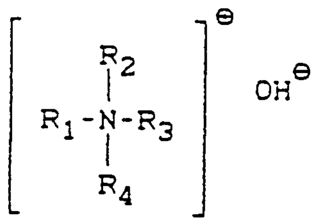

für welche
$R_1$, $R_2$ und $R_3$ für gleiche oder verschiedene Alkylreste mit 1 bis 18 Kohlenstoffatomen und
$R_4$ für einen Benzyl-, 2-Hydroxyethyl-, 2-Hydroxypropyl- oder 2-Hydroxybutyl-Rest
steht, als Trimerisierungskatalysator,
Beendigung der Trimerisierungsreaktion beim angestrebten Trimerisierungsgrad durch Zugabe eines Katalysatorengifts und/oder thermische Desaktivierung des Katalysators und Entfernung von nicht umgesetztem Hexamethylendiisocyanat bis auf einen Pestgehalt von max. 0,2 Gew.-%, dadurch gekennzeichnet, daß man

a) das als Ausgangsmaterial zum Einsatz gelangende Hexamethylendiisocyanat von Kohlendioxid bis auf einen Restgehalt von weniger als 20 ppm (Gewicht) befreit und

b) den Katalysator in einer Menge von weniger als 0,03 Gew.-%, bezogen auf das Gewicht des eingesetzten Hexamethylendiisocyanats, verwendet.

2. Verfahren nach Anspruch 1, dadurch gekennzeichnet, daß man das als Ausgangsmaterial zum Einsatz gelangende Hexamethylendiisocyanat von Kohlendioxid bis auf einen Restgehalt von weniger als 10 ppm (Gewicht) befreit.

3. Verfahren gemäß Anspruch 1 und 2, dadurch gekennzeichnet, daß man den Katalysator in einer Menge von weniger als 0,01 Gew.-%, bezogen auf das Gewicht des eingesetzten Hexamethylendiisocyanats, verwendet.

4. Verfahren nach Anspruch 1 bis 3, dadurch gekennzeichnet, daß man als Katalysator N,N,N-Trimethyl-N-benzylammoniumhydroxid verwendet.

5. Verfahren gemäß Anspruch 1 bis 3, dadurch gekennzeichnet, daß man als Katalysator N,N,N-Trimethyl-N-(2-hydroxyethyl)-ammoniumhydroxid verwendet.

6. Verfahren nach Anspruch 1 bis 3, dadurch gekennzeichnet, daß man als Katalysator N,N,N-Trimethyl-N-(2-hydroxypropyl)-ammoniumhydroxid verwendet.

7. Verfahren gemäß Anspruch 1 bis 6, dadurch gekennzeichnet, daß man Dibutylphosphat als Katalysatorengift verwendet.

8. Verfahren nach Anspruch 1 bis 6, dadurch gekennzeichnet, daß man Di-(2-ethylhexyl)-phosphat als Katalysatorengift verwendet.

## Claims

1. A process for the production of polyisocyanates containing isocyanurate groups by partial trimerization of the isocyanate groups in hexamethylene diisocyanate using guaternary ammonium hydroxides corresponding to the following formula

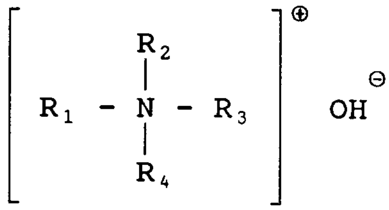

in which
$R_1$, $R_2$ and $R_3$ may be the same or different and represent $C_{1-18}$ alkyl radicals and
$R_4$ is a benzyl, 2-hydroxyethyl, 2-hydroxypropyl or 2-hydroxybutyl radical,
as trimerization catalyst,
termination of the trimerization reaction at the desired degree of trimerization by addition of a catalyst poison and/or thermal deactivation of the catalyst and removal of unreacted hexamethylene diisocyanate to a residual content of at most 0.2% by weight, characterized in that

a) the hexamethylene diisocyanate used as starting material is freed from carbon dioxide to a residual content of less than 20 ppm (weight) and
b) the catalyst is used in a quantity of less than 0.03% by weight, based on the weight of the hexamethylene diisocyanate used.

**2.** A process as claimed in claim 1, characterized in that the hexamethylene diisocyanate used as starting material is freed from carbon dioxide to a residual content of less than 10 ppm (weight).

**3.** A process as claimed in claims 1 and 2, characterized in that the catalyst is used in a quantity of less than 0.01% by weight, based on the weight of the hexamethylene diisocyanate used.

**4.** A process as claimed in claims 1 to 3, characterized in that N,N,N-trimethyl-N-benzyl ammonium hydroxide is used as the catalyst.

**5.** A process as claimed in claims 1 to 3, characterized in that N,N,N-trimethyl-N-(2-hydroxyethyl)-ammonium hydroxide is used as the catalyst.

**6.** A process as claimed in claims 1 to 3, characterized in that N,N,N-trimethyl-N-(2-hydroxypropyl)-ammonium hydroxide is used as the catalyst.

**7.** A process as claimed in claims 1 to 6, characterized in that dibutyl phosphate is used as the catalyst poison.

**8.** A process as claimed in claims 1 to 6, characterized in that di-(2-ethylhexyl)-phosphate is used as the catalyst poison.

## Revendications

**1.** Procédé pour la préparation de polyisocyanates présentant des groupes isocyanurate par trimérisation d'une partie des groupes isocyanate de l'hexaméthylènediisocyanate en utilisant des hydroxydes d'ammonium quaternaires répondant à la formule générale

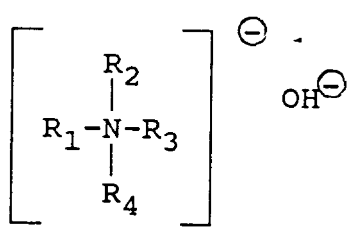

dans laquelle
$R_1$, $R_2$ et $R_3$ représentent des radicaux alkyle identiques ou différents contenant de 1 à 18 atomes de carbone, et
$R_4$ représente un radical benzyle, un radical 2-hydroxyéthyle, un radical 2-hydroxypropyle ou un radical 2-hydroxybutyle,
comme catalyseur de trimérisation,
en mettant un terme à la réaction de trimérisation après avoir atteint le degré de trimérisation recherché, par addition d'un poison de catalyseur et/ou par désactivation thermique du catalyseur et élimination de l'hexaméthylènediisocyanate n'ayant pas réagi, jusqu'à une teneur résiduelle de maximum 0,2% en poids, caractérisé en ce que

a) on libère du dioxyde de carbone, l'hexaméthylènediisocyanate à mettre en oeuvre comme matière de départ, jusqu'à une teneur résiduelle inférieure à 20 ppm (en poids), et

b) on utilise le catalyseur en une quantité inférieure à 0,03% en poids, rapportée au poids de l'hexaméthylènediisocyanate mis en oeuvre.

**2.** Procédé selon la revendication 1, caractérisé en ce qu'on libère du dioxyde de carbone, l'hexaméthylènediisocyanate à mettre en oeuvre comme matière de départ, jusqu'à une teneur résiduelle inférieure à 10 ppm (en poids).

**3.** Procédé selon les revendications 1 et 2, caractérisé en ce qu'on utilise le catalyseur en une quantité inférieure à 0,01% en poids, rapportée au poids de l'hexaméthylènediisocyanate mis en oeuvre.

**4.** Procédé selon la revendication 1 à 3, caractérisé en ce qu'on utilise, comme catalyseur, l'hydroxyde de N,N,N-triméthyl-N-benzylammonium.

**5.** Procédé selon les revendications 1 à 3, caractérisé en ce qu'on utilise, comme catalyseur, l'hydroxyde de N,N,N-triméthyl-N-(2-hydroxyéthyl) ammonium.

**6.** Procédé selon les revendications 1 à 3, caractérisé en ce qu'on utilise, comme catalyseur, l'hydroxyde de N,N,N-triméthyl-N-(2-hydroxypropyl) ammonium.

**7.** Procédé selon les revendications 1 à 6, caractérisé en ce qu'on utilise le phosphate de dibutyle comme poison de catalyseurs.

**8.** Procédé selon les revendications 1 à 6, caractérisé en ce qu'on utilise le di-(2-éthylhexyl)phosphate comme poison de catalyseurs.